(19) Europäisches Patentamt
European Patent Office
Office européen des brevets

(11) Veröffentlichungsnummer : **0 314 003 B1**

(12) # EUROPÄISCHE PATENTSCHRIFT

(45) Veröffentlichungstag der Patentschrift : **21.08.91 Patentblatt 91/34**

(51) Int. Cl.$^5$ : **C07C 15/40**, C07C 1/24, C07B 35/06

(21) Anmeldenummer : **88117538.4**

(22) Anmeldetag : **21.10.88**

(54) **Verfahren zur Herstellung von 1-Aryl-alk-1-enen.**

(30) Priorität : **30.10.87 DE 3736819**

(43) Veröffentlichungstag der Anmeldung : **03.05.89 Patentblatt 89/18**

(45) Bekanntmachung des Hinweises auf die Patenterteilung : **21.08.91 Patentblatt 91/34**

(84) Benannte Vertragsstaaten : **BE CH DE ES FR GB IT LI NL**

(56) Entgegenhaltungen : **FR-A- 2 401 126**

(73) Patentinhaber : **BASF Aktiengesellschaft
Carl-Bosch-Strasse 38
W-6700 Ludwigshafen (DE)**

(72) Erfinder : **Himmele, Walter Dr.,
Eichenweg 14
W-6909 Walldorf (DE)**
Erfinder : **Bott, Kaspar Dr.,
Werderstrasse 57
W-6800 Mannheim 1 (DE)**
Erfinder : **Bronstert, Klaus, Dr.
Gartenstrasse 26
W-6719 Calsberg (DE)**

**EP 0 314 003 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von 1-Aryl-alk-1-enen durch Dehydratisierung von 1-Aryl-alkan-1-olen in Gegenwart eines wasserabspaltenden Mittels.

Für die Herstellung der 1-Aryl-alk-1-ene aus den entsprechenden Aryl-alkan-1-olen sind bereits mehrere Methoden beschrieben. Eine umfassende Zusammenstellung der Dehydratisierungsverfahren findet sich in Houben-Weyl, Methoden der organischen Chemie, Band V/1b, S. 45-62, S. 70-104, Georg Thieme Verlag Stuttgart 1972.

Die Dehydratisierung läßt sich nach diesen Angaben sowohl in der flüssigen als auch in der Gasphase durchführen.

Als Katalysatoren für die Wasserabspaltung in der flüssigen Phase werden sowohl saure als auch basische Verbindungen genannt.

Als saure Katalysatoren werden z.B. Schwefelsäure, para-Toluolsulfonsäure, Phosphorsäure, Borsäure, Ameisensäure, ferner Acetanhydrid, Phthalsäureanhydrid und Verbindungen wie Kaliumhydrogensulfat und Kupfersulfat empfohlen.

Diese sauren Substanzen katalysieren zwar im allgemeinen die Wasserabspaltung von 1-Aryl-alkan-1-olen sehr gut, jedoch haben sie auch deutliche Nachteile. Bei der Verwendung von starken Säuren kommt es zur Bildung von beachtlichen Anteilen an Nebenprodukten, weil die entstehenden Olefine z.B. miteinander unter Ausbildung von linearen Polymeren oder auch unter Alkylierung der aromatischen Ringe weiterreagieren.

Ferner besteht bei den bisher angewandten, stark sauren Reaktionsbedingungen die Gefahr von protonenkatalysierten Doppelbindungsisomerisierungen und Alkylgruppenwanderungen. Tragen die Edukte säurelabile Substituenten, wie z.B. Ethergruppen, so geben diese zu weiteren Nebenreaktionen Anlaß.

Weiterhin ist bei Anwendung dieser Katalysatoren die unmittelbare destillative Isolierung des Produktes aus der Reaktionsmischung praktisch nicht möglich. Statt dessen ist eine kontinuierliche, mehrstufige Aufarbeitung der Reaktionsmischung, nämlich Neutralisation, Extraktion sowie Destillation, notwendig.

Außerdem führt der Einsatz mancher der genannten Katalysatoren zu Ablagerungen und Verkrustungen an den Wandungen der Reaktionsgefäße, deren Entfernung aufwendige Reinigungsmaßnahmen erfordert.

Einige der bisher verwendeten Katalysatoren binden das gebildete Reaktionswasser, so daß dieses nicht destillativ aus der Reaktionsmischung abgetrennt werden kann. Da die Menge des gebildeten Reaktionswassers ein Maß für den Grad der Umsetzung ist, wäre es jedoch wünschenswert, das Reaktionswasser laufend abdestillieren zu können.

Der Erfindung lag deshalb die Aufgabe zugrunde, ein Verfahren zur Dehydratisierung von Aryl-alkan-1-olen zu 1-Ary-alk-1-enen zu finden, das, ohne mit Nachteilen der bisherigen Verfahren behaftet zu sein, es zusätzlich erlaubt, das gebildete Reaktionswasser laufend aus dem Reaktionsgemisch abzudestillieren und das insbesondere die unmittelbare, destillative Isolierung des Produkts ohne weitere Aufarbeitung ermöglicht.

Demgemäß wurde ein Verfahren zur Herstellung von 1-Aryl-alk-1-enen durch Dehydratisierung von 1-Aryl-alken-1-olen in Gegenwart eines wasserabspaltenden Mittels gefunden, das dadurch gekennzeichnet ist, daß man als wasserabspaltendes Mittel ein mit mindestens einer Arylgruppe verestertes Triphosphit verwendet.

Das erfindungsgemäße Verfahren läßt sich durch folgende allgemeine Reaktionsgleichung wiedergeben:

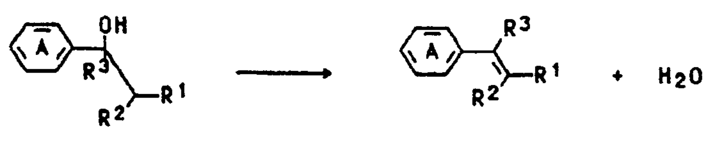

I II

$R^1$, $R^2$ und $R^3$ sind dabei Wasserstoff, wobei einer der Reste von Wasserstoff verschieden ist, oder unter den Reaktionsbedingungen inerte Substituenten. Inerte Substituenten sind z.B. Aryl- und im wesentlichen Alkylgruppen, die jedoch noch weiter durch Cycloalkyl, Aryl oder Alkoxy substituiert sein können. Als Substituenten für die Arylgruppen sowie den Ring A kommen z.B. Alkyl, Alkoxy, Fluor, Chlor oder Brom oder anellierte Ringe in Betracht.

Bevorzugt sind für $R^1$, $R^2$ und $R^3$ z.B. Wasserstoff oder lineares oder verzweigtes $C_1$- bis $C_{18}$-Alkyl, das noch durch Phenyl substituiert sein kann. Bevorzugte Substituenten für den Ring A sind z.B. Fluor oder Chlor. Alkylreste sind dabei z.B. Methyl, Ethyl, n- oder i-Propyl, n- oder i-Hexyl, β-Ethylhexyl, Octyl, Decyl, Dodecyl oder Hexadecyl.

Die erfindungsgemäß verwendeten Arylphosphite sind Ester der phosphorigen Säure der allgemeinen Formel III

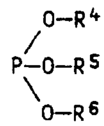

III

in der $R^4$, $R^5$ und $R^6$ gleich oder verschieden sein können und einen Aryl-, einen $C_1$- bis $C_{20}$-Alkyl-, einen $C_7$- bis $C_{20}$-Aralkyl- und/oder einen $C_3$- bis $C_8$-Cycloalkylrest bedeuten, mit der Maßgabe, daß mindestens einer der Reste $R^4$, $R^5$ oder $R^6$ ein Arylrest ist.

Als wasserabspaltende Phosphite sind insbesondere Di- und Triarylphosphite, wie Diphenyl-alkyl- oder Triphenylphosphit, wobei die Phenylreste noch durch Alkyl substituiert sein können, geeignet. Beispielsweise genannt seien Diphenyl-n-hexyl-, Diphenyl-cyclohexyl-, Triphenyl- oder Tritolylphosphit, wobei die letztgenannten bevorzugt sind.

Die Phosphite werden vorzugsweise in katalytischen Mengen, z.B. 0,05 bis 2%, insbesondere 0,2 bis 1%, bezogen auf das Gewicht der Reaktionsmischung verwendet.

Für die Dehydratisierung geeignete Temperaturen liegen im allgemeinen im Bereich von 120 bis 225°C, vorzugsweise 140 bis 200°C. Bei Reaktionszeiten von einigen Stunden erreicht man dabei in der Regel Umsätze von mehr als 90%.

Als besonders zweckmäßig hat es sich erwiesen, das bei der Reaktion entstehende Wasser durch ein Schleppmittel destillativ zu entfernen, bei dem nach der Entmischung das Wasser die untere Phase bildet; durch die Menge des abgeschiedenen Wassers hat man gleichzeitig ein Maß für den erzielten Umsatz.

Die Reaktion kann nach Erzielen des erwünschten Umsatzes abgebrochen werden, um eine unnötige thermische Belastung des Olefins zu vermeiden.

Als Schleppmittel für die Entfernung des Wassers eignen sich z.B. Alkane mittlerer C-Zahl oder alkylsubstituierte Aromaten wie Toluol, Xylol oder Diisopropylbenzol.

Das Reaktionsgemisch wird nach beendeter Dehydratisierung zweckmäßigerweise durch fraktionierte Destillation aufgetrennt. Diese Fraktionierung kann direkt, d.h. ohne weitere Vorbehandlung vorgenommen werden. In den Beispielen ist das erfindungsgemäße Verfahren in weiteren Einzelheiten beschrieben.

Die zur Herstellung der erfindungsgemäßen 1-Aryl-alk-1-ene benötigten 1-Aryl-alkan-1-ole können entweder durch Grignard-Reaktion (s. Organikum, S. 618ff, VEB Deutscher Verlag der Wissenschaften, Berlin 1976) aus den jeweiligen Halogenverbindungen und den entsprechenden Aldehyden bzw. Ketonen dargestellt werden (Verbindungen der Tabellen 1 bis 5) oder aus den entsprechenden Alkoholen mit Hilfe der Guerbet-Reaktion (s. EP-A 0227057) synthetisiert werden (Verbindungen der Tabelle 6). Die erfindungsgemäß herstellbaren Olefine sind wertvolle Zwischenprodukte für bei der Polymerisation von anionisch polymerisierbaren Monomeren notwendige Initiatoren (s. dazu z.B. DE-A-3729144).

Beispiel 1

Herstellung von 1-(p-Chlorphenyl)-oct-1-en durch Dehydratisierung von 1-(p-Chlorphenyl)-octan-1-ol

In einem 2-l-Rührkolben, der mit einem Wasserabscheider, Tropftrichter und Thermometer ausgestattet war, wurden 1 109 g (4,60 Mol) 1-(p-Chlorphenyl)-octan-1-ol (I) vorgelegt. Als Dehydratisierungskatalysator wurden 10 g Triphenylphosphit zugegeben (0,9%). Zum Ausschleppen des bei der Reaktion gebildeten Wassers wurde ein Gemisch von n-Octan und Xylol verwendet. Die Temperatur im Rührkolben wurde auf 194 bis 196°C eingestellt.

Im Verlauf von 5 Stunden wurden 67 g Wasser aus der Vorlage des Wasserabscheiders entnommen. Nach Zugabe von weiteren 5 g Triphenylphosphit (1,35%) konnten nach weiteren 5 Stunden, während derer die Temperatur zwischen 193 bis 198°C gehalten wurde, noch 8 g Wasser erhalten werden. Die insgesamt erhaltene Wassermenge von 75 g (4,16 Mol) entspricht 90,6% der zu erwartenden Menge an Wasser.

Die Isolierung und Reinigung des 1-(p-Chlorphenyl)-oct-1-ens wurde durch Fraktionierung des nicht weiter behandelten Reaktionsgemisches aus der Dehydratisierung über eine Siebboden-Kolonne mit 10 Siebböden vorgenommen. Bei einem Druck von 20 mbar destillierten im Temperaturbereich von 130 bis 132°C 919 g 1-(p-Chlorphenyl)-oct-1-en (II) über. Die Anfangsfraktionen enthielten geringe Anteile an Z-Isomerem. Etwa 92% des Olefins II fielen als E-Isomeres an. Die Ausbeute an II bezogen auf den Alkohol betrug 89,6%.

Beispiel 2

Herstellung von 1-(p-Fluorphenyl)-3,5,5-trimethyl-hex-1-en (III) durch Dehydratisierung von 1-(p-Fluorphenyl)-3,5,5-trimethyl-hexan-1-ol (IV).

350 g IV wurden mit 5 g Tri-o-tolyl-phosphit bei 168 bis 184°C mit Hilfe eines n-Oktan-Xylol-Gemischs (1 : 2) im Verlauf von 10 Stunden dehydratisiert. Es wurden 22 g Wasser erhalten ; das entspricht 83.7% der nach der Theorie zu erwartenden Menge.

Die Fraktionierung des nicht weiter behandelten Reaktionsgemischs über eine Siebbodenkolonne mit 10 Siebböden lieferte 282 g (1,28 Mol) Olefin III. III destilliert bei einem Druck von 8 mbar zwischen 110 bis 114°C über. Die Anfangsfraktionen enthielten geringe Anteile an Z-isomerem Olefin III. Etwa 90% des Olefins III fiellen in der E-Form an. Die Ausbeute bezogen auf den nicht speziell gereinigten Alkohol IV, der durch Grignard-Reaktion aus p-Fluor-brombenzol und 3,5,5-Trimethyl-hexanal synthetisiert worden war, betrug 87,2%.

Beispiele 1 bis 48 in den Tabellen 1 bis 5

In der Tabelle 1 sind erfindungsgemäß hergestellte fluor-substituierte 1-Aryl-alk-1-ene aufgelistet. Die Darstellung der fluorsubstituierten 1-Aryl-alkan-1-ole erfolgte durch Grignard-Reaktion ausgehend von 4-Fluorbenzaldehyd oder 4-Fluor-brombenzol mit entsprechenden Aldehyden und Ketonen bzw. Halogenverbindungen. Sämtliche Ausgangsverbindungen sind bekannt und können zum größten Teil käuflich erworben werden. 4-Phenyl-4-methylvaleraldehyd, der als Ausgangsmaterial zur Herstellung von Verbindung 6 dient, wird aus dem entsprechenden Alken (s. EP-A 0219092) durch Hydroformylierung (s. Houben-Weyl, Methoden der Organischen Chemie, 4. Auflage, Bd. 7/1, Thieme, Stuttgart) gewonnen. 3,5,5-Trimethylcapronaldehyd — Ausgangsstoff zur Synthese von Verbindung 10 — wird durch Hydroformylierung von Diisobutylen nach Houben-Weyl, Methoden der Organischen Chemie, 4. Auf., Bd. 7/1, S. 58, Thieme, Stuttgart, erhalten.

In der Tabelle 2 sind chlor-substituierte 1-Aryl-alk-1-ene aufgeführt. Die Synthese dieser Verbindungen beruht auf der Grignard-Reaktion der bekannten Verbindungen 2-Chlor- bzw. 4-Chlorbenzaldehyd mit den entsprechenden Halogenverbindungen zu den entsprechenden Arylalkanolen.

Aus Brombenzol, 4-Methyl-brombenzol, 4-Methoxy-brombenzol und 4-Fluor-brombenzol werden ebenfalls durch Grignard-Reaktion mit den entsprechenden Aldehyden bzw. Ketonen die zur Herstellung der 1-Aryl-alk-1-ene der Tabellen 3, 4 und 5 benötigten Arylalkanole gewonnen.

Die in der Tabelle 6 genannten Olefine wurden aus entsprechenden 1-Aryl-alkan-1-olen hergestellt, welche mit Hilfe der Guerbet-Reaktion aus bekannten Ausgangsstoffen erhältlich sind.

Die erzielten (nicht optimierten) Ausbeuten sind jeweils mit der Angabe des Siedebereichs genannt. Die Ausbeuten beziehen sich auf die Aryl-1-alkan-1-ole.

Tabelle 1

Synthese von 1-(4-Fluorphenyl)-alk-1-en auf Basis von 4-Fluorbenzaldehyd (1) bzw. 4-Fluor-brom-benzol (2)

| Strukturformel | Nr. | Ausbeute [%] | physik. Stoffdaten |
|---|---|---|---|
| 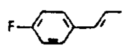 | 1 | 85 | $Kp_{10}$ 65- 66°C |
| 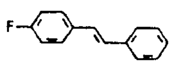 | 2 | 82,0 | $Kp_2$ 126°C |
| 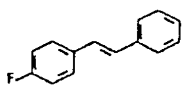 | 3 | | Kp 108-113°C |
| 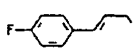 | 4 | 78 | $Kp_{28}$ 95°C |
| 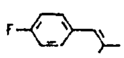 | 5 | 61 | $Kp_{23}$ 80°C |
| 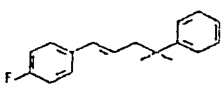 | 6 | 72 | $Kp_2$ 140°C |
| 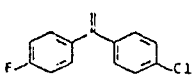 | 7 | | $Kp_1$ 108°C |

Tabelle 1: Fortsetzung

| Strukturformel | Nr. | Ausbeute [%] | physik. Stoffdaten |
|---|---|---|---|
| 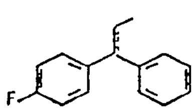 | 8 | 91 | $Kp_3$ 87°C |
| 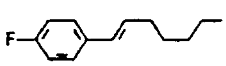 | 9 | 87,8 | $Kp_2$ 87°C |
| 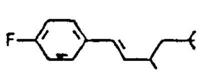 | 10 | 87,2 | $Kp_2$ 110-114°C |

Tabelle 2

Synthese von 1-(4-Chlor-phenyl)-1-alkenen auf Basis von 4-Chlorbenzaldehyd

| Strukturformel | Nr. | Ausbeute [%] | physik. Stoffdaten |
|---|---|---|---|
| 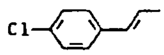 | 11 | 80,0 | $Kp_{20}$ = 100°C |
| 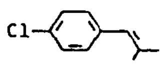 | 12 | 69,0 | $Kp_{30}$ 117°C |
| 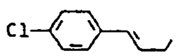 | 13 | 69,5 | $Kp_{2}$ 71-72°C |
| 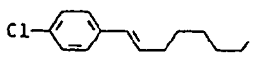 | 14 | 89,6 | $Kp_{2}$ 130°C |
| 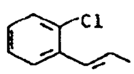 | 15 | 77,0 | $K_{32}$ 121-122°C |
| 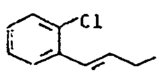 | 16 | 71,7 | $Kp_{30}$ 120°C |
| 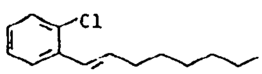 | 17 | 75,8 | $Kp_{2}$ 103°C |
| 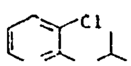 | 18 | | $Kp_{2}$ 76-82°C |

Tabelle 3

Synthese von 1-Aryl-alk-1-enen auf Basis von Brom-benzol und speziellen Rhodium-oxo-aldehyden

| Strukturformel | Nr. | Ausbeute [%] | physik. Stoffdaten |
|---|---|---|---|
| 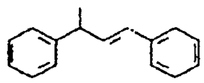 | 19 | 97,0 | $Kp_4$ 138-142°C |
| 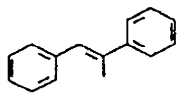 | 20 | 93,0 | $Kp_2$ 108-116°C |
| 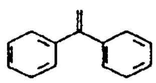 | 21 | 95,5 | $Kp_2$ 98-106°C |
| 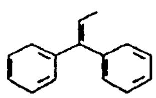 | 22 | 90,2 | $Kp_2$ 104-108°C |
| 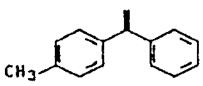 | 23 | 95,8 | $Kp_2$ 120-124°C |
| 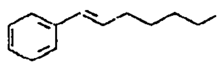 | 24 | 81,8 | $Kp_2$ 75°C |
| 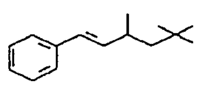 | 25 | 60,3 | $Kp_2$ 77°C |

Tabelle 3: Fortsetzung

| Strukturformel | Nr. | Ausbeute [%] | physik. Stoffdaten |
|---|---|---|---|
| 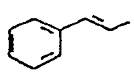 | 26 | | Kp 117-119°C |
| 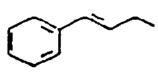 | 27 | | $Kp_{22}$ 84°C |

Tabelle 4

Synthese von 1,1-Di-phenyl-prop-1-enen auf Basis von Propiophenon

| Strukturformel | Nr. | Ausbeute [%] | physik. Stoffdaten |
|---|---|---|---|
| 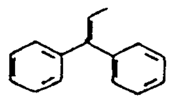 | 28 | 90,0 | $Kp_2$ 100-106°C |
| 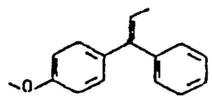 | 29 | | $Kp_2$ 138°C |
| 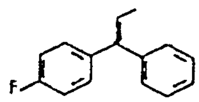 | 30 | | $Kp_2$ 105°C |
| 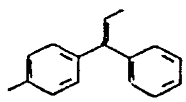 | 31 | 85,3 | $Kp_2$ 107°C |
| 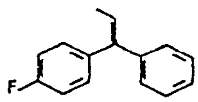 | 32 | | $Kp_2$ 97-98°C |

Tabelle 5

Synthese von 1,1-Diphenyl-ethenen

| Strukturformel | Nr. | Ausbeute [%] | physik. Stoffdaten |
|---|---|---|---|
| 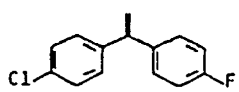 | 33 | 92,8 | $Kp_2$ 106-108°C |
| 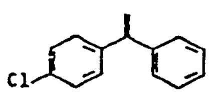 | 34 | 95,7 | $Kp_1$ 118-124°C |
| 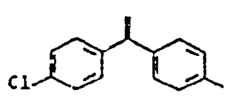 | 35 | 88,6 | $Kp_1$ 128-132°C |

Tabelle 6

Synthese von 1-Aryl-alk-1-enen mit Hilfe von 1-Aryl-alkan-1-olen auf Basis der Guerbet-Reaktion

| Strukturformel | Nr. | Ausbeute [%] | physik. Stoffdaten |
|---|---|---|---|
| 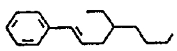 | 36 | 90,0 | $Kp_2$ 115-118°C |
| 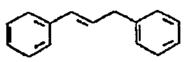 | 37 | 88,0 | $Kp_2$ 111-112°C |
| 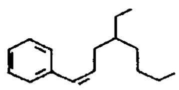 | 38 | | $Kp_4$ 128-130°C |
| 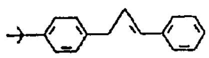 | 39 | | $KP_2$ 146-147°C |
| 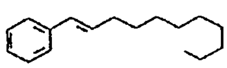 | 40 | | $Kp_2$ 104°C |
| 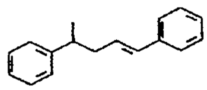 | 41 | | $Kp_2$ 123-124°C |
| 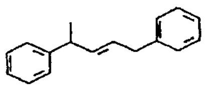 | 42 | | $Kp_2$ 125°C |

Tabelle 6: Fortsetzung

| Strukturformel | Nr. | Ausbeute [%] | physik. Stoffdaten |
|---|---|---|---|
| 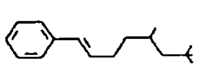 | 43 | | $Kp_{18}$ 167-168°C |
| 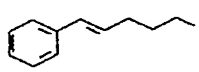 | 44 | 90,4 | $K_{80}$ 146-148°C |
| 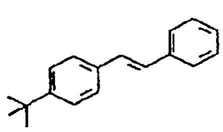 | 45 | 88,7 | Fp 99-101°C |
| 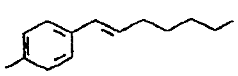 | 47 | | $Kp_4$ 78-79°C |
| 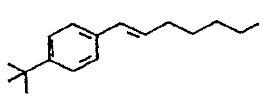 | 48 | 92 | $Kp_2$ 124°C°C |

## Patentansprüche

1. Verfahren zur Herstellung von 1-Aryl-alk-1-enen durch Dehydratisierung von 1-Aryl-alkan-1-olen in Gegenwart eines wasserabspaltenden Mittels, dadurch gekennzeichnet, daß man als wasserabspaltendes Mittel ein mit mindestens einer Arylgruppe verestertes Triphosphit verwendet.

2. Verfahren nach Anspruch 1, dadurch gekennzeichnet, daß man als wasserabspaltendes Mittel ein Triarylphosphit verwendet.

3. Verfahren nach Anspruch 1, dadurch gekennzeichnet, daß man als wasserabspaltendes Mittel Triphenylphosphit oder Tritolylphosphit verwendet.

4. Verfahren nach Anspruch 1, dadurch gekennzeichnet, daß man das bei der Reaktion entstehende Wasser mit Hilfe eines Schleppmittels entfernt.

5. Verfahren nach Anspruch 1, dadurch gekennzeichnet, daß man nach beendeter Wasserabspaltung zur Gewinnung der 1-Aryl-alk-1-ene das Reaktionsgemisch ohne weitere Reinigungsoperation fraktioniert destilliert.

## Claims

1. A process for preparing 1-aryl-1-alkenes by dehydration of 1-aryl-1-alkanols in the presence of a dehydrating agent, which comprises using as dehydrating agent a triphosphite esterified with at least one aryl group.

2. A process as claimed in claim 1, wherein a triaryl phosphite is used as dehydrating agent.

3. A process as claimed in claim 1, wherein triphenyl phosphite or tritolyl phosphite is used as dehydrating agent.

4. A process as claimed in claim 1, wherein the water produced in the reaction is removed with the aid of an entraining agent.

5. A process as claimed in claim 1, wherein the reaction mixture after completion of dehydration is fractionally distilled, without further purification operation, to obtain the 1-aryl-1-alkenes.

## Revendications

1. Procédé de préparation de 1-arylalcènes-1 par déshydratation de 1-arylalcanols-1 en présence d'un déshydratant, caractérisé en ce qu'on utilise comme déshydratant un triphosphite estérifié avec au moins un groupement aryle.

2. Procédé selon la revendication 1, caractérisé en ce qu'on utilise comme déshydratant un phosphite de triaryle.

3. Procédé selon la revendication 1, caractérisé en ce qu'on utilise comme déshydratant le phosphite de triphényle ou le phosphite de tritolyle.

4. Procédé selon la revendication 1, caractérisé en ce qu'on chasse l'eau formée par la réaction à l'aide d'un agent d'entraînement.

5. Procédé selon la revendication 1, caractérisé en ce que, une fois la déshydratation terminée, on effectue une distillation fractionnée du mélange réactionnel sans autre opération de purification, pour récupérer le 1-arylalcène-1.